(19) Europäisches Patentamt
European Patent Office
Office européen des brevets

(11) **EP 3 779 727 A1**

(12) **EUROPEAN PATENT APPLICATION**
published in accordance with Art. 153(4) EPC

(43) Date of publication:
**17.02.2021 Bulletin 2021/07**

(51) Int Cl.:
***G06F 16/35*** (2019.01)

(21) Application number: **19795972.9**

(22) Date of filing: **23.04.2019**

(86) International application number:
**PCT/JP2019/017191**

(87) International publication number:
**WO 2019/212005 (07.11.2019 Gazette 2019/45)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(30) Priority: **02.05.2018 JP 2018088828**

(71) Applicant: **Fronteo, Inc.**
**Tokyo 108-0075 (JP)**

(72) Inventors:
• **TOYOSHIBA, Hiroyoshi**
**Tokyo 108-0075 (JP)**
• **UCHIYAMA, Hidefumi**
**Tokyo 108-0075 (JP)**

(74) Representative: **Vossius & Partner**
**Patentanwälte Rechtsanwälte mbB**
**Siebertstrasse 3**
**81675 München (DE)**

(54) **DANGEROUS BEHAVIOR PREDICTION DEVICE, PREDICTION MODEL GENERATION DEVICE, AND DANGEROUS BEHAVIOR PREDICTION PROGRAM**

(57) Included are a learning data input unit 10 that inputs m texts included in the medical information of patient, a similarity index value computation unit 100 that extracts n words from m texts and computes a similarity index value reflecting a relationship between the m texts and the n words, a classification model generation unit 14 that generates a classification model for classifying m texts into a plurality of phenomena based on a text index value group including n similarity index values for one text, and an unsafe incident prediction unit 21 that predicts a possibility of occurrence of falling from a text to be predicted by applying a similarity index value computed by the similarity index value computation unit 100 from a text input by a prediction data input unit 20 to a classification model, and a highly accurate classification model is generated using a similarity index value that represents which word contributes to which text and to what extent.

FIG. 1

EP 3 779 727 A1

**Description**

Technical Field

The present invention relates to an unsafe incident prediction device, a prediction model generation device, and an unsafe incident prediction program, and particularly relates to a technology for predicting a possibility that a patient performs unsafe incident such as falling or tumbling, and a technology for generating a prediction model used for this prediction.

Background Art

In recent years, prevention of incidents has been emphasized in various industrial fields. In a medical field, various measures are being considered to prevent medical accidents. For example, there is provided a system for preventing medical accidents by recording incident reports and managing unsafe actions that may lead to medical accidents based on the incident reports.

Incidentally, the medical accidents include an accident caused by medical treatment of a doctor, a nurse, etc. and an accident caused by a situation on the patient side, for example, falling of the patient. While it is possible to prevent the former accident as much as possible by improving the quality of the medical treatment by the doctor, the nurse, etc., it is difficult to prevent the latter accident, which has a large factor on the patient side, in the first place. Therefore, in the conventional measures, it is an actual situation that only rough measures such as uniformly regulating the behavior of the patient can be taken.

Note that Patent Document 1 discloses an apparatus that predicts an unsafe incident (falling, tumbling, etc.) of a patient. In the behavior prediction apparatus described in Patent Document 1, medical information associated with the unsafe incident extracted in advance from decided medical record information that is medical record information in which the unsafe incident is specified by being linked to an incident report related to the unsafe incident of the patient is stored in a storage unit. A relationship evaluation unit acquires undecided medical record information to which the incident report is not linked, and evaluates a relationship between the undecided medical record information and the unsafe incident that may be performed by the patient corresponding to the undecided medical record information based on the medical information associated with the unsafe incident stored in the storage unit. A prediction unit predicts the unsafe incident of the patient corresponding to the undecided medical record information according to an evaluation result of the relationship evaluation unit.

Specifically, the behavior prediction apparatus described in Patent Document 1 associates emotion evaluations for data elements included in the medical record information (data elements including emotional expressions of the patient, for example, morpheme such as "easy", "sore", and "painful") and stores the emotion evaluations in the storage unit. In addition, the behavior prediction apparatus searches textual matter included in the medical record information to determine whether a predetermined keyword (word related to an emotion) is included in the textual matter. Then, when the predetermined keyword is included, an emotion score computed according to a predetermined standard is associated with the keyword and stored in the storage unit.

Meanwhile, the behavior prediction apparatus extracts a keyword related to a predetermined emotion from undecided medical record information, acquires an emotion score associated with the extracted keyword from the storage unit, and integrates emotion scores of respective keywords, thereby obtaining an emotion score of the undecided medical record information. For example, it is presumed that text "I have a pain in my leg recently. I flutter when I stand up" is included in textual matter of the undecided medical record information. Further, it is presumed that "pain" and "flutter" are stored in advance in the storage unit as keywords, and emotion scores of "+1.4" and "+0.9" are associated with the keywords, respectively. In this case, the behavior prediction apparatus computes an emotion score of "+2.3" by adding the scores. Then, the behavior prediction apparatus predicts an unsafe incident (falling) of the patient based on the emotion score.

Citation List

Patent Document

Patent Document 1: Japanese Patent No. 5,977,898

Summary of the Invention

Technical Problem

In the case of predicting falling by machine learning as in the above-mentioned Patent Document 1, it is necessary to improve accuracy of a prediction model generated by learning to improve accuracy of prediction. However, in the behavior prediction apparatus described in the above-mentioned Patent Document 1, the score used for prediction is simply calculated based on a degree at which a predetermined keyword related to emotion is included in medical record information, and a prediction model generated accordingly is an extremely simple one in which the computed score is associated with the keyword and stored. For this reason, there is a problem that it is difficult to sufficiently improve the accuracy of prediction.

The invention has been made to solve such a problem, and an object of the invention is to make it possible to accurately predict occurrence of unsafe incident caused by a person such as falling or tumbling by analyzing a text included in medical information such as an electronic medical record.

Solution to Problem

To solve the above-mentioned problem, in an unsafe incident prediction device of the invention, m texts included in medical information related to a patient for whom it is known whether the patient has performed unsafe incident are input as learning data, the input m texts are analyzed to extract n words from the m texts, each of the m texts is converted into a q-dimensional vector according to a predetermined rule, thereby computing m text vectors including q axis components, and each of the n words is converted into a q-dimensional vector according to a predetermined rule, thereby computing n word vectors including q axis components. Further, each of the inner products of the m text vectors and the n word vectors is taken to compute $m \times n$ similarity index values reflecting a relationship between the m texts and the n words. Then, a classification model for classifying m texts for a degree of possibility of occurrence of unsafe incident is generated based on a text index value group including n similarity index values per one text. At the time of predicting a possibility of occurrence of unsafe incident for a patient corresponding to a prediction target, m' texts included in medical information related to a patient corresponding to a prediction target are input as prediction data, and a similarity index value obtained by executing each process of word extraction, text vector computation, word vector computation, and index value computation on the input prediction data is applied to a classification model, thereby predicting a possibility that the patient corresponding to the prediction target performs unsafe incident.

Advantageous Effects of the Invention

According to the invention configured as described above, since an inner product of a text vector computed from a text included in the medical information of patient and a word vector computed from a word included in the text is calculated to compute a similarity index value reflecting a relationship between the text and the word, it is possible to obtain which word contributes to which text and to what extent, or which text contributes to which word and to what extent as an inner product value. Further, since a classification model is generated using a similarity index value having such a characteristic, it is possible to appropriately classify a text corresponding to each patient for a degree of possibility of occurrence of unsafe incident, taking into account a level of contribution of m texts and n words. Therefore, according to the invention, in an apparatus for predicting a possibility that a patient performs unsafe incident, it is possible to increase accuracy of a classification model generated by learning to accurately predict occurrence of unsafe incident.

Brief Description of the Drawings

Fig. 1 is a block diagram illustrating a functional configuration example of an unsafe incident prediction device according to an embodiment.
Fig. 2 is a flowchart illustrating an operation example of the unsafe incident prediction device according to the embodiment.
Fig. 3 is a block diagram illustrating another functional configuration example of an unsafe incident prediction device according to an embodiment.

Mode for Carrying Out the Invention

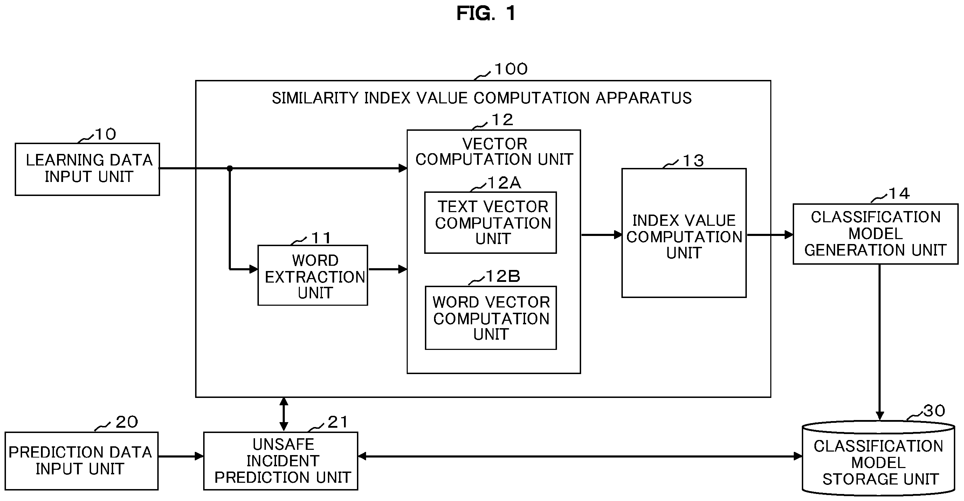

An embodiment of the invention will be described below with reference to the drawings. Fig. 1 is a block diagram illustrating a functional configuration example of an unsafe incident prediction device according to the embodiment. As a functional configuration, the unsafe incident prediction device of the present embodiment includes a learning data input unit 10, a word extraction unit 11, a vector computation unit 12, an index value computation unit 13, a classification model generation unit 14, a prediction data input unit 20, and an unsafe incident prediction unit 21. The vector computation unit 12 includes a text vector computation unit 12A and a word vector computation unit 12B as a more specific functional configuration. Further, the unsafe incident prediction device of the present embodiment includes a classification model storage unit 30 as a storage medium.

Note that for the sake of convenience of the following description, a part including the word extraction unit 11, the vector computation unit 12, and the index value computation unit 13 will be referred to as a similarity index value computation unit 100. The similarity index value computation unit 100 inputs text data related to a text, and computes and outputs a similarity index value that reflects a relationship between the text and a word contained therein. In addition, the unsafe incident prediction device of the present embodiment uses a similarity index value computed by the similarity index value computation unit 100 analyzing a text included in an electronic medical record (corresponding to medical information in the claims) of a patient to predict a possibility that the patient performs unsafe incident (for example, falling during walking or bathing, or tumbling from a bed or a toilet seat, which will be simply referred to as falling or tumbling below) from content of the text included in the electronic medical record. Note that the prediction model generation device of the invention includes the learning data input unit 10, the similarity index value computation unit 100, and the classification model generation unit 14.

Each of the functional blocks 10 to 14 and 20 to 21 can be configured by any of hardware, a Digital Signal Processor (DSP), and software. For example, in the case of being configured by software, each of the functional blocks 10 to 14 and 20 to 21 actually includes a CPU, a RAM, a ROM, etc. of a computer, and is implemented by operation of a program stored in a recording medium such as a RAM, a ROM, a hard disk, or a semiconductor memory.

The learning data input unit 10 inputs m texts (m is an arbitrary integer of 2 or more) included in an electronic medical record related to a patient for whom it is known whether the patient has performed unsafe incident of falling or tumbling as learning data. For example, the learning data input unit 10 inputs an electronic medical record of a past inpatient for whom presence or absence of occurrence of falling or tumbling during hospitalization is reported in description of the electronic medical record or another report, and inputs a text having medical record textual matter included in the electronic medical record as learning data.

The electronic medical record includes a department, a consultation date, medical record textual matter, etc. in addition to personal information of the patient such as name, date of birth, blood type, and gender. The learning data input unit 10 inputs the electronic medical record in a state where a part of the medical record textual matter in the electronic medical record set to be used as learning data (strictly speaking, the electronic medical record is input to use a text of the medical record textual matter in the electronic medical record as learning data). Note that the text of the medical record textual matter input by the learning data input unit 10, that is, a text to be analyzed described below may include one sentence (unit divided by a period) or include a plurality of sentences.

The word extraction unit 11 analyzes m texts input by the learning data input unit 10, and extracts n words (n is an arbitrary integer of 2 or more) from the m texts. As a text analysis method, for example, a known morphological analysis can be used. Here, the word extraction unit 11 may extract morphemes of all parts of speech divided by morphological analysis as words, or may extract only morphemes of specific parts of speech as words.

Note that m texts may include a plurality of the same words. In this case, the word extraction unit 11 does not extract a plurality of the same words, and extracts only one word. That is, n words extracted by the word extraction unit 11 refer to n types of words. Here, the word extraction unit 11 may measure a frequency with which the same word is extracted from m texts in the electronic medical record, and extract n words (n types) in a descending order of the appearance frequency or n words (n types) whose appearance frequency is greater than or equal to a threshold value.

The vector computation unit 12 computes m text vectors and n word vectors from m texts and n words. Here, the text vector computation unit 12A converts each of the m texts targeted for analysis by the word extraction unit 11 into a q-dimensional vector according to a predetermined rule, thereby computing m text vectors including q (q is an arbitrary integer of 2 or more) axis components. In addition, the word vector computation unit 12B converts each of the n words extracted by the word extraction unit 11 into a q-dimensional vector according to a predetermined rule, thereby computing n word vectors including q axis components.

In the present embodiment, as an example, a text vector and a word vector are computed as follows. Now, a set $S = <d \in D, w \in W>$ including the m texts and the n words is considered. Here, a text vector $d_i \rightarrow$ and a word vector $W_j \rightarrow$ (hereinafter, the symbol "$\rightarrow$" indicates a vector) are associated with each text $d_i$ (i = 1, 2, ..., m) and each word $W_j$ (j = 1, 2, ..., n), respectively. Then, a probability $P(w_j|d_i)$ shown in the following Equation (1) is calculated with respect to an arbitrary word $W_j$ and an arbitrary text $d_i$.

[Equation 1]

$$P(w_j \mid d_i) = \frac{\exp(\vec{w}_j \cdot \vec{d}_i)}{\sum_{k=1}^{n} \exp(\vec{w}_k \cdot \vec{d}_i)} \quad \cdots (1)$$

Note that the probability $P(W_j|d_i)$ is a value that can be computed in accordance with a probability p disclosed in, for example, a follow thesis describing evaluation of a text or a document by a paragraph vector. "'Distributed Representations of Sentences and Documents' by Quoc Le and Tomas Mikolov, Google Inc; Proceedings of the 31st International Conference on Machine Learning Held in Bejing, China on 22-24 June 2014" This thesis states that, for example, when there are three words "the", "cat", and "sat", "on" is predicted as a fourth word, and a computation formula of the prediction probability p is described. The probability p(wt|wt - k, ..., wt + k) described in the thesis is a correct answer probability when another word wt is predicted from a plurality of words wt - k, ..., wt + k.

Meanwhile, the probability $P(w_j|d_i)$ shown in Equation (1) used in the present embodiment represents a correct answer probability that one word $w_j$ of n words is predicted from one text di of m texts. Predicting one word $w_j$ from one text di means that, specifically, when a certain text $d_i$ appears, a possibility of including the word $w_j$ in the text di is predicted.

In Equation (1), an exponential function value is used, where e is the base and the inner product of the word vector w→ and the text vector d→ is the exponent. Then, a ratio of an exponential function value calculated from a combination of a text di and a word $w_j$ to be predicted to the sum of n exponential function values calculated from each combination of the text $d_i$ and n words $w_k$ (k = 1, 2, ..., n) is calculated as a correct answer probability that one word $w_j$ is expected from one text $d_i$.

Here, the inner product value of the word vector $W_j$→ and the text vector $d_i$→ can be regarded as a scalar value when the word vector $W_j$→ is projected in a direction of the text vector $d_i$→, that is, a component value in the direction of the text vector $d_i$→ included in the word vector $w_j$→, which can be considered to represent a degree at which the word $w_j$ contributes to the text $d_i$. Therefore, obtaining the ratio of the exponential function value calculated for one word $W_j$ to the sum of the exponential function values calculated for n words $w_k$ (k = 1, 2, ..., n) using the exponential function value calculated using the inner product corresponds to obtaining the correct answer probability that one word $w_j$ of n words is predicted from one text $d_i$.

Note that since Equation (1) is symmetrical with respect to di and $w_j$, a probability $P(d_i|w_j)$ that one text $d_i$ of m texts is predicted from one word $w_j$ of n words may be calculated. Predicting one text di from one word $w_j$ means that, when a certain word $w_j$ appears, a possibility of including the word $w_j$ in the text di is predicted. In this case, an inner product value of the text vector $d_i$→ and the word vector $w_j$→ can be regarded as a scalar value when the text vector $d_i$→ is projected in a direction of the word vector $w_j$→, that is, a component value in the direction of the word vector $W_j$→ included in the text vector $d_i$→, which can be considered to represent a degree at which the text di contributes to the word $w_j$.

Note that here, a calculation example using the exponential function value using the inner product value of the word vector w→ and the text vector d→ as an exponent has been described. However, the exponential function value may not be used. Any calculation formula using the inner product value of the word vector w→ and the text vector d→ may be used. For example, the probability may be obtained from the ratio of the inner product values.

Next, the vector computation unit 12 computes the text vector $d_i$→ and the word vector $w_j$→ that maximize a value L of the sum of the probability $P(w_j|d_i)$ computed by Equation (1) for all the set S as shown in the following Equation (2). That is, the text vector computation unit 12A and the word vector computation unit 12B compute the probability $P(w_j|d_i)$ computed by Equation (1) for all combinations of the m texts and the n words, and compute the text vector $d_i$→ and the word vector $w_j$→ that maximize a target variable L using the sum thereof as the target variable L.

[Equation 2]

$$L = \sum_{d \in D} \sum_{w \in W} \#(w, d)\, p(w \mid d) \quad \cdots (2)$$

Maximizing the total value L of the probability $P(w_j|d_i)$ computed for all the combinations of the m texts and the n words corresponds to maximizing the correct answer probability that a certain word $w_j$ (j = 1, 2, ..., n) is predicted from a certain text $d_i$ (i = 1, 2, ..., m). That is, the vector computation unit 12 can be considered to compute the text vector $d_i$→ and the word vector $w_j$→ that maximize the correct answer probability.

Here, in the present embodiment, as described above, the vector computation unit 12 converts each of the m texts di into a q-dimensional vector to compute the m texts vectors $d_i$→ including the q axis components, and converts each of the n words into a q-dimensional vector to compute the n word vectors $w_j$→ including the q axis components, which corresponds to computing the text vector $d_i$→ and the word vector $w_j$→ that maximize the target variable L by making q axis directions variable.

The index value computation unit 13 takes each of the inner products of the m text vectors $d_i$→ and the n word

vectors $w_j{\rightarrow}$ computed by the vector computation unit 12, thereby computing $m \times n$ similarity index values reflecting the relationship between the $m$ texts $d_i$ and the $n$ words $w_j$. In the present embodiment, as shown in the following Equation (3), the index value computation unit 13 obtains the product of a text matrix D having the respective q axis components ($d_{11}$ to $d_{mq}$) of the $m$ text vectors $d_i{\rightarrow}$ as respective elements and a word matrix W having the respective q axis components ($w_{11}$ to $w_{nq}$) of the $n$ word vectors $w_j{\rightarrow}$ as respective elements, thereby computing an index value matrix DW having $m \times n$ similarity index values as elements. Here, $W^t$ is the transposed matrix of the word matrix.

[Equation 3]

$$D = \begin{pmatrix} d_{11} & d_{12} & \cdots & d_{1q} \\ d_{21} & d_{22} & \cdots & d_{2q} \\ \vdots & \vdots & \ddots & \vdots \\ d_{m1} & d_{m2} & \cdots & d_{mq} \end{pmatrix} \qquad W = \begin{pmatrix} w_{11} & w_{12} & \cdots & w_{1q} \\ w_{21} & w_{22} & \cdots & w_{2q} \\ \vdots & \vdots & \ddots & \vdots \\ w_{n1} & w_{m2} & \cdots & w_{mq} \end{pmatrix}$$

$$DW = D * W^t = \begin{pmatrix} dw_{11} & dw_{12} & \cdots & dw_{1n} \\ dw_{21} & dw_{22} & \cdots & dw_{2n} \\ \vdots & \vdots & \ddots & \vdots \\ dw_{m1} & dw_{m2} & \cdots & dw_{mn} \end{pmatrix} \qquad \cdots (3)$$

Each element of the index value matrix DW computed in this manner may indicate which word contributes to which text and to what extent. For example, an element $dw_{12}$ in the first row and the second column is a value indicating a degree at which the word $w_2$ contributes to a text $d_1$. In this way, each row of the index value matrix DW can be used to evaluate the similarity of a text, and each column can be used to evaluate the similarity of a word.

The classification model generation unit 14 generates a classification model for classifying $m$ respective texts di into two ranks for a degree of possibility of occurrence of falling or tumbling based on a text index value group including $n$ similarity index values $dw_j$ ($j$ = 1, 2, ..., n) per one text $d_i$ ($i$ = 1, 2, ..., m) using $m \times n$ similarity index values computed by the index value computation unit 13. That is, the classification model generation unit 14 generates a classification model in which classification into "falling or tumbling occurs" is performed for a text index value group computed based on an electronic medical record of a patient for whom it is known that the patient fell or tumbled, and classification into "no falling or tumbling" is performed for a text index value group computed based on an electronic medical record of a patient for whom it is known that the patient has not fell or tumbled. Then, the classification model generation unit 14 causes the classification model storage unit 30 to store the generated classification model.

Here, for example, in the case of a first text $d_1$, $n$ similarity index values $dw_{11}$ to $dw_{1n}$ included in a first row of the index value matrix DW correspond to a text index value group. Similarly, in the case of a second text $d_2$, $n$ similarity index values $dw_{21}$ to $dw_{2n}$ included in a second row of the index value matrix DW correspond to a text index value group. Hereinafter, this description is similarly applied to text index value groups up to a text index value group ($n$ similarity index values $dw_{m1}$ to $dw_{mn}$) related to an mth text $d_m$.

For example, the classification model generation unit 14 generates a classification model for classifying the respective texts $d_i$ into two phenomena by computing each feature quantity for a text index value group of each text $d_i$, and optimizing two-group separation by the Markov chain Monte Carlo method according to a value of the computed feature quantity. Here, the classification model generated by the classification model generation unit 14 is a learning model that uses a text index value group as an input and outputs one of the two phenomena desired to be predicted (presence or absence of a possibility of occurrence of falling or tumbling) as a solution. Alternatively, it is possible to adopt a learning model that outputs, as a numerical value, a probability of being classified as "possibility of falling or tumbling is present". A form of the learning model is arbitrary.

For example, a form of the classification model generated by the classification model generation unit 14 may be set to any one of a regression model (learning model based on linear regression, logistic regression, support vector machine, etc.), a tree model (learning model based on decision tree, regression tree, random forest, gradient boosting tree, etc.), a neural network model (learning model based on perceptron, convolutional neural network, recurrent neural network, residual network, RBF network, stochastic neural network, spiking neural network, complex neural network, etc.), a Bayesian model (learning model based on Bayesian inference), a clustering model (learning model based on k-nearest neighbor method, hierarchical clustering, non-hierarchical clustering, topic model, etc.), etc. Note that the classification models listed here are merely examples, and the invention is not limited thereto.

The prediction data input unit 20 inputs m' texts (m' is an arbitrary integer of 1 or more) included in an electronic medical record related to a patient to be predicted as prediction data. For example, the prediction data input unit 20 inputs electronic medical records as many as the number of current inpatients in a hospital in which the unsafe incident prediction device of the present embodiment is introduced, and inputs texts having medical record textual matter included in the electronic medical records as prediction data.

In an actual operation of the hospital, it is preferable that the prediction data input unit 20 periodically (for example, every day) inputs the electronic medical record of each inpatient, and the unsafe incident prediction unit 21 regularly predicts falling or tumbling of each inpatient. For example, the prediction data input unit 20 may periodically input the electronic medical record of each inpatient from an electronic medical record system (not illustrated) that saves data of the electronic medical record. Description of the medical record textual matter in the electronic medical record may be updated through daily medical treatment by a doctor. Therefore, based on content of the text of the medical record textual matter that may be updated, falling or tumbling of each inpatient is predicted on a daily basis.

Here, the electronic medical records input by the prediction data input unit 20 are set to electronic medical records of a patient having an unknown possibility of occurrence of falling or tumbling and a patient currently predicted to have no possibility of occurrence of falling or tumbling. An electronic medical record of a patient previously predicted to have a possibility of occurrence of falling or tumbling may not be an input target. However, since improvement of a symptom or a physical condition of a patient may eliminate the possibility of occurrence of falling or tumbling, the electronic medical record of the patient previously predicted to have the possibility of occurrence of falling or tumbling may be an input target.

Note that a database in which an update history of the electronic medical record and a prediction execution history of falling or tumbling are recorded for each patient may be created, and the prediction data input unit 20 may selectively input an electronic medical record of a patient corresponding to a prediction target from an electronic medical record system based on history information of this database. For example, the prediction data input unit 20 may search for an electronic medical record of a patient whose history information indicates that the electronic medical record is updated and a process of predicting falling or tumbling is not executed after the update from the electronic medical record system and input the electronic medical record.

The unsafe incident prediction unit 21 predicts a possibility that a patient corresponding to a prediction target performs unsafe incident such as falling or tumbling by applying a similarity index value obtained by executing processing of the word extraction unit 11, the vector computation unit 12, and the index value computation unit 13 of the similarity index value computation unit 100 for prediction data input by the prediction data input unit 20 to a classification model generated by the classification model generation unit 14 (classification model stored in the classification model storage unit 30).

For example, when m' texts of medical record textual matter included in the electronic medical record are input by the prediction data input unit 20 as prediction data, m' text index value groups are obtained by executing processing of the similarity index value computation unit 100 for the m' texts of the medical record textual matter according to an instruction of the unsafe incident prediction unit 21. The unsafe incident prediction unit 21 applies the m' text index value groups computed by the similarity index value computation unit 100 to the classification model as input data one by one, thereby predicting the possibility of occurrence of falling or tumbling of the patient for each of the m' texts.

Here, it is preferable that the word extraction unit 11 extracts the same words as n words extracted from m pieces of learning data from prediction data. A reason is that since a text index value group including n words extracted from prediction data has the same words as those of a text index value group including n words extracted from learning data as elements, conformity to a classification model stored in the classification model storage unit 30 increases. However, it is not necessary to extract, at the time of prediction, the same n words as those at the time of learning since in a case where a text index value group for prediction is generated by a combination of words different from those at the time of learning, even though conformity to the classification model decreases, it is possible to predict a possibility of corresponding to a phenomenon using the fact that conformity is low as an element of evaluation.

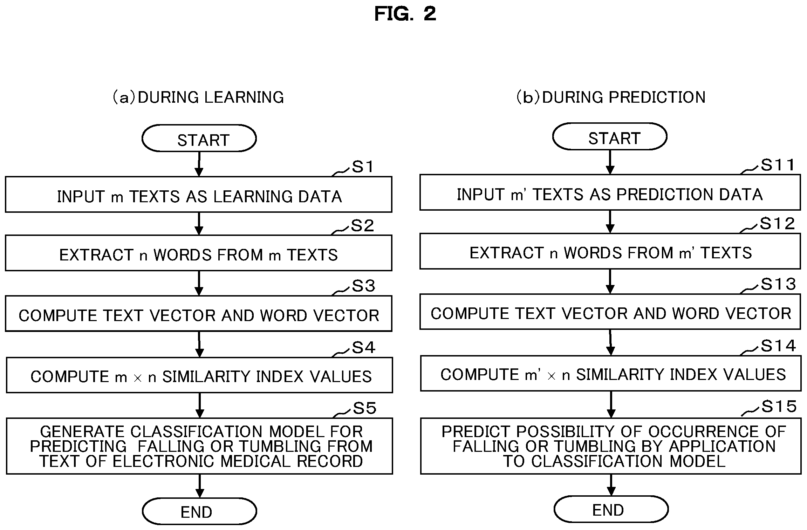

Fig. 2 is a flowchart illustrating an operation example of the unsafe incident prediction device according to the present embodiment configured as described above. Fig. 2(a) illustrates an operation example during learning for generating a classification model, and Fig. 2(b) illustrates an operation example during prediction for predicting the possibility of occurrence of falling or tumbling using the generated classification model.

During learning illustrated in Fig. 2(a), first, the learning data input unit 10 inputs m texts (medical record textual matter) included in an electronic medical record related to a patient for whom it is known whether the patient has performed unsafe incident of falling or tumbling as learning data (step S1). The word extraction unit 11 analyzes the m texts input by the learning data input unit 10, and extracts n words from the m texts (step S2).

Subsequently, the vector computation unit 12 computes m text vectors $d_i\rightarrow$ and n word vectors $w_j\rightarrow$ from the

m texts input by the learning data input unit 10 and the n words extracted by the word extraction unit 11 (step S3). Then, the index value computation unit 13 obtains each of the inner products of the m text vectors $d_i\rightarrow$ and the n word vectors $w_j\rightarrow$, thereby computing m × n similarity index values (index value matrix DW having m × n similarity index values as respective elements) reflecting a relationship between the m texts $d_i$ and the n words $w_j$ (step S4).

Further, the classification model generation unit 14 generates a classification model for classifying the m texts di into two ranks for a degree of possibility of occurrence of falling or tumbling based on a text index value group including n similarity index values $dw_j$ per one text di using the m × n similarity index values computed by the index value computation unit 13, and causes the classification model storage unit 30 to store the generated classification model (step S5). In this way, the operation during learning ends.

During prediction illustrated in Fig. 2(b), first, the prediction data input unit 20 inputs m' texts (medical record textual matter) included in an electronic medical record related to a patient corresponding to a prediction target as prediction data (step S11). The unsafe incident prediction unit 21 supplies the prediction data input by the prediction data input unit 20 to the similarity index value computation unit 100, and gives an instruction to compute a similarity index value.

According to this instruction, the word extraction unit 11 analyzes the m' texts input by the prediction data input unit 20, and extracts n words from the m' texts (the same words as those extracted from the learning data) (step S12). Note that not all the n words may be included in the m' texts. A null value is given for a word not existing in the m' texts.

Subsequently, the vector computation unit 12 computes m' text vectors $d_i\rightarrow$ and n word vectors $w_j\rightarrow$ from the m' texts input by the prediction data input unit 20 and the n words extracted by the word extraction unit 11 (step S13).

Then, the index value computation unit 13 obtains each of the inner products of the m' text vectors $d_i\rightarrow$ and the n word vectors $w_j\rightarrow$, thereby computing m' × n similarity index values (index value matrix DW having m' × n similarity index values as respective elements) reflecting a relationship between the m' texts di and the n words $w_j$ (step S14). The index value computation unit 13 supplies the computed m' × n similarity index values to the unsafe incident prediction unit 21.

The unsafe incident prediction unit 21 predicts a possibility that the patient corresponding to the prediction target performs the unsafe incident of falling or tumbling for each of the m' texts by applying each of m' text index value groups to a classification model stored in the classification model storage unit 30 based on the m' × n similarity index values supplied from the similarity index value computation unit 100 (step S15). In this way, the operation during prediction ends.

As described in detail above, in the present embodiment, the m texts included in the electronic medical record of the patient are input as learning data, the inner product of a text vector computed from the input text and a word vector computed from a word included in the text is calculated to compute a similarity index value reflecting a relationship between the text and the word, and a classification model is generated using this similarity index value. In this way, a classification model is generated using the similarity index value representing which word contributes to which text and to what extent, or which text contributes to which word and to what extent. For this reason, it is possible to appropriately classify a text in the electronic medical record into one of two phenomena of the presence or absence of the possibility of occurrence of falling or tumbling, taking into account a level of contribution of the m texts and the n words. Therefore, according to the present embodiment, in an apparatus for predicting a possibility that a patient performs unsafe incident, it is possible to increase accuracy of a classification model generated by learning to accurately predict occurrence of unsafe incident.

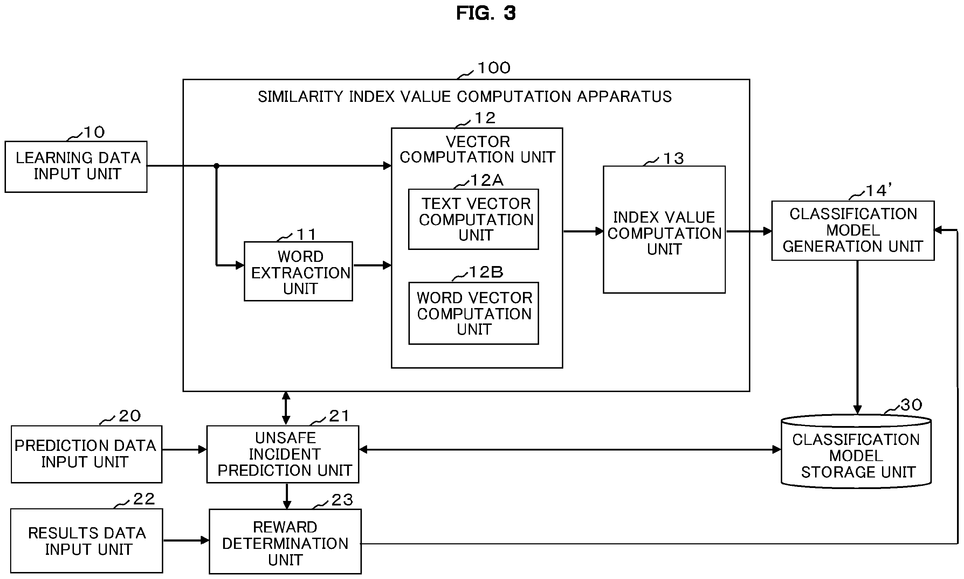

Note that in the present embodiment, a description has been given of an example of applying supervised learning that uses text data related to a text that is known in terms of which one of the two phenomena of "falling or tumbling occurs" and "no falling or tumbling" a phenomenon to which the text corresponds is, as learning data. Above supervised learning may be combined with reinforcement learning. Fig 3 is a block diagram illustrating a functional configuration example of an unsafe incident prediction device according to another embodiment in which a mechanism for reinforcement learning is added.

As illustrated in Fig. 3, the unsafe incident prediction device according to another embodiment further includes a results data input unit 22 and a reward determination unit 23 in addition to the configuration illustrated in Fig. 1. In addition, the unsafe incident prediction device according to another embodiment includes a classification model generation unit 14' instead of the classification model generation unit 14 illustrated in Fig. 1.

The results data input unit 22 inputs an unsafe incident recording report included in an electronic medical record of a discharged patient as results data. That is, the electronic medical record may include items of a post-discharge summary in addition to the name, the date of birth, the blood type, the gender, the department, the consultation date, and the medical record textual matter of the patient described above. This post-discharge summary is an item for describing a condition of the patient during hospitalization as a summary after the discharge of the patient. A recording report of whether the patient has performed unsafe incident during hospitalization is described in this post-discharge summary. The results data input unit 22 inputs content of the unsafe incident recording report described in the post-discharge summary, that is, information on whether or not the patient has performed unsafe incident during hospitalization as results data.

Note that a method of inputting the results data by the results data input unit 22 is not limited thereto. For example, information on whether or not the patient has performed unsafe incident during hospitalization may be described in the medical record textual matter of the electronic medical record. Therefore, the results data input unit 22 may input content of the unsafe incident recording report described in the medical record textual matter as results data.

Specifically, the results data input unit 22 determines whether a patient has performed unsafe incident during hospitalization by analyzing a text described in a post-discharge summary or medical record textual matter, and inputs a determination result as results data. Alternatively, the presence or absence of the occurrence of unsafe incident during hospitalization of the discharged patient may be input as the results data by a medical worker such as a doctor or a nurse visually confirming a text described in the post-discharge summary or the medical record textual matter, and the results data input unit 22 inputting information input by the medical worker such as the doctor or the nurse operating an input device such as a keyboard or a touch panel.

The reward determination unit 23 determines a reward to be given to the classification model generation unit 14' according to results of occurrence of falling or tumbling input by the results data input unit 22 with respect to a possibility of occurrence of falling or tumbling predicted by the unsafe incident prediction unit 21. For example, the reward determination unit 23 determines to give a positive reward when prediction data indicating the possibility of occurrence of falling or tumbling predicted by the unsafe incident prediction unit 21 matches the results data input by the results data input unit 22, and determines to give no reward or negative reward when the prediction data does not match the results data.

Similarly to the classification model generation unit 14 illustrated in Fig. 1, the classification model generation unit 14' generates a classification model based on learning data input by the learning data input unit 10, and causes the classification model storage unit 30 to store the generated classification model. In addition, the classification model generation unit 14' modifies the classification model stored in the classification model storage unit 30 according to a reward determined by the reward determination unit 23. As described above, by adding a mechanism of reinforcement learning to a mechanism of supervised learning to generate the classification model, it is possible to further improve the accuracy of the classification model.

In the embodiment, a description has been given of an example of using an electronic medical record as medical information used for learning and prediction. However, for example, medical information other than the electronic medical record may be used as long as a text such as a nursing record report that can predict a possibility of occurrence of unsafe incident of a patient is included.

In the embodiment, a description has been given of an example in which a possibility of occurrence of falling or tumbling is predicted as unsafe incident of a patient. However, the invention is not limited thereto. That is, the invention can be widely used to predict occurrence of unsafe incident resulting from a situation on the patient side rather than the doctor or nurse side.

In the embodiment, a description has been given of an example in which texts are classified into two ranks for a degree of possibility of occurrence of falling or tumbling. However, the texts may be classified into three or more ranks.

In the embodiment, a description has been given of predicting occurrence of falling or tumbling related to a hospitalized patient. However, the invention is not limited thereto. For example, it is possible to predict a possibility of occurrence of falling or tumbling at home for a patient having an electronic medical record or similar medical information, such as an outpatient, a patient targeted for home-visit treatment, or a teletherapy patient using a telemedicine system.

In addition, in the embodiment, a description has been given of predicting a possibility that a patient performs unsafe incident in a hospital. However, it is possible to predict a possibility that a care recipient performs unsafe incident in a long-term care facility, etc. In the scope of this specification and claims, it is presumed that the care recipient is a concept included in the "patient".

In addition, the embodiment is merely an example of a specific embodiment for carrying out the invention, and the technical scope of the invention should not be interpreted in a limited manner. That is, the invention can be implemented in various forms without departing from the gist or the main features thereof.

Reference Signs List

| | |
|---|---|
| 10 | Learning data input unit |
| 11 | Word extraction unit |
| 12 | Vector computation unit |
| 12A | Text vector computation unit |
| 12B | Word vector computation unit |
| 13 | Index value computation unit |

14, 14'    Classification model generation unit
20    Prediction data input unit
21    Unsafe incident prediction unit
22    Results data input unit
23    Reward determination unit
30    Classification model storage unit
100    Similarity index value computation unit

**Claims**

1. An unsafe incident prediction device **characterized by** comprising:

    a learning data input unit that inputs m texts (m is an arbitrary integer of 2 or more) included in medical information related to a patient for whom it is known whether the patient has performed unsafe incident as learning data;
    a word extraction unit that analyzes the m texts input by the learning data input unit as the learning data, and extracts n words (n is an arbitrary integer of 2 or more) from the m texts;
    a text vector computation unit that converts each of the m texts into a q-dimension vector (q is an arbitrary integer of 2 or more) according to a predetermined rule, thereby computing m text vectors including q axis components;
    a word vector computation unit that converts each of the n words into a q-dimension vector according to a predetermined rule, thereby computing n word vectors including q axis components;
    an index value computation unit that takes each of inner products of the m text vectors and the n word vectors, thereby computing $m \times n$ similarity index values reflecting a relationship between the m texts and the n words;
    a classification model generation unit that uses the $m \times n$ similarity index values computed by the index value computation unit to generate a classification model for classifying the m texts for a degree of possibility of occurrence of the unsafe incident based on a text index value group including n similarity index values per one text;
    a prediction data input unit that inputs m' texts (m' is an arbitrary integer of 1 or more) included in medical information related to a patient corresponding to a prediction target as prediction data; and
    an unsafe incident prediction unit that predicts a possibility that the patient corresponding to the prediction target performs unsafe incident by applying a similarity index value obtained by executing processing of the word extraction unit, the text vector computation unit, the word vector computation unit and the index value computation unit for the prediction data input by the prediction data input unit to the classification model generated by the classification model generation unit.

2. The unsafe incident prediction device according to claim 1, **characterized in that** the text vector computation unit and the word vector computation unit set, to a target variable, a value obtained by computing and adding a probability that one of the m texts is expected from one of the n words, or a probability that one of the n words is expected from one of the m texts for all combinations of the m texts and the n words, and compute a text vector and a word vector for maximizing the target variable.

3. The unsafe incident prediction device according to claim 1 or 2, **characterized in that** the index value computation unit calculates a product of a text matrix having the respective q axis components of the m text vectors as respective elements and a word matrix having the respective q axis components of the n word vectors as respective elements, thereby computing an index value matrix having the $m \times n$ similarity index values as respective elements.

4. The unsafe incident prediction device according to any one of claims 1 to 3,
    wherein the learning data input unit inputs an electronic medical record of a patient for whom it is known whether the patient has performed unsafe incident as the medical information, and inputs a text having medical record textual matter included in the electronic medical record as the learning data, and
    the prediction data input unit inputs an electronic medical record of a current inpatient as the medical information, and inputs a text having medical record textual matter included in the electronic medical record as the prediction data.

5. The unsafe incident prediction device according to claim 4, further comprising:

    a results data input unit that inputs an unsafe incident recording report included in an electronic medical record of a discharged patient as results data; and

EP 3 779 727 A1

a reward determination unit that determines a reward to be given to the classification model generation unit according to occurrence results of the unsafe incident indicated by the results data input by the results data input unit with respect to a possibility of occurrence of the unsafe incident predicted by the unsafe incident prediction unit during hospitalization of the discharged patient,
wherein the classification model generation unit modifies the classification model according to a reward determined by the reward determination unit.

6. A prediction model generation device **characterized by** comprising:

a learning data input unit that inputs m texts (m is an arbitrary integer of 2 or more) included in medical information related to a patient for whom it is known whether the patient has performed unsafe incident as learning data;
a word extraction unit that analyzes the m texts input by the learning data input unit as the learning data, and extracts n words (n is an arbitrary integer of 2 or more) from the m texts;
a text vector computation unit that converts each of the m texts into a q-dimension vector (q is an arbitrary integer of 2 or more) according to a predetermined rule, thereby computing m text vectors including q axis components;
a word vector computation unit that converts each of the n words into a q-dimension vector according to a predetermined rule, thereby computing n word vectors including q axis components;
an index value computation unit that takes each of inner products of the m text vectors and the n word vectors, thereby computing m $\times$ n similarity index values reflecting a relationship between the m texts and the n words;
a classification model generation unit that uses the m $\times$ n similarity index values computed by the index value computation unit to generate a classification model for classifying the m texts for a degree of possibility of occurrence of the unsafe incident as a prediction model for predicting a possibility of occurrence of the unsafe incident from the texts based on a text index value group including n similarity index values per one text.

7. The prediction model generation device according to claim 6, **characterized in that** the text vector computation unit and the word vector computation unit compute a probability that one of the m texts is predicted from one of the n words or a probability that one of the n words is predicted from one of the m texts for all combinations of the m texts and the n words, set a total value thereof as a target variable, and compute a text vector and a word vector maximizing the target variable.

8. The prediction model generation device according to claim 6 or 7, **characterized in that** the index value computation unit calculates a product of a text matrix having the respective q axis components of the m text vectors as respective elements and a word matrix having the respective q axis components of the n word vectors as respective elements, thereby computing an index value matrix having the m $\times$ n similarity index values as respective elements.

9. An unsafe incident prediction program causing a computer to function as:

a learning data input means that inputs m texts (m is an arbitrary integer of 2 or more) included in medical information related to a patient for whom it is known whether the patient has performed unsafe incident as learning data;
a word extraction unit means that analyzes the m texts input by the learning data input means as the learning data, and extracts n words (n is an arbitrary integer of 2 or more) from the m texts;
a vector computation means that converts each of the m texts into a q-dimension vector (q is an arbitrary integer of 2 or more) according to a predetermined rule and converts each of the n words into a q-dimension vector according to a predetermined rule, thereby computing m text vectors including q axis components and n word vectors including q axis components;
an index value computation means that takes each of inner products of the m text vectors and the n word vectors, thereby computing m $\times$ n similarity index values reflecting a relationship between the m texts and the n words; and
classification model generation means that uses the m $\times$ n similarity index values computed by the index value computation means to generate a classification model for classifying the m texts for a degree of possibility of occurrence of the unsafe incident as a prediction model for predicting possibility of occurrence of the unsafe incident from the texts based on a text index value group including n similarity index values per one text.

10. The unsafe incident prediction program according to claim 9, further causing a computer to function as:

a prediction data input means that inputs m' texts (m' is an arbitrary integer of 1 or more) included in medical information related to a patient corresponding to a prediction target as prediction data; and
an unsafe incident prediction means that predicts a possibility that the patient corresponding to the prediction target performs unsafe incident by applying a similarity index value obtained by executing processing of the word extraction means, the text vector computation means, the word vector computation means and the index value computation means for the prediction data input by the prediction data input means to the classification model generated by the classification model generation means.

**FIG. 1**

SIMILARITY INDEX VALUE COMPUTATION APPARATUS ~100

LEARNING DATA INPUT UNIT ~10

WORD EXTRACTION UNIT ~11

VECTOR COMPUTATION UNIT ~12

TEXT VECTOR COMPUTATION UNIT ~12A

WORD VECTOR COMPUTATION UNIT ~12B

INDEX VALUE COMPUTATION UNIT ~13

CLASSIFICATION MODEL GENERATION UNIT ~14

PREDICTION DATA INPUT UNIT ~20

UNSAFE INCIDENT PREDICTION UNIT ~21

CLASSIFICATION MODEL STORAGE UNIT ~30

# FIG. 2

(a)DURING LEARNING

START

↓ S1

INPUT m TEXTS AS LEARNING DATA

↓ S2

EXTRACT n WORDS FROM m TEXTS

↓ S3

COMPUTE TEXT VECTOR AND WORD VECTOR

↓ S4

COMPUTE m × n SIMILARITY INDEX VALUES

↓ S5

GENERATE CLASSIFICATION MODEL FOR PREDICTING FALLING OR TUMBLING FROM TEXT OF ELECTRONIC MEDICAL RECORD

↓

END

(b)DURING PREDICTION

START

↓ S11

INPUT m' TEXTS AS PREDICTION DATA

↓ S12

EXTRACT n WORDS FROM m' TEXTS

↓ S13

COMPUTE TEXT VECTOR AND WORD VECTOR

↓ S14

COMPUTE m' × n SIMILARITY INDEX VALUES

↓ S15

PREDICT POSSIBILITY OF OCCURRENCE OF FALLING OR TUMBLING BY APPLICATION TO CLASSIFICATION MODEL

↓

END

EP 3 779 727 A1

## FIG. 3

EP 3 779 727 A1

<table>
<tr><td colspan="2" align="center">**INTERNATIONAL SEARCH REPORT**</td><td colspan="2">International application No.<br>PCT/JP2019/017191</td></tr>
</table>

A.  CLASSIFICATION OF SUBJECT MATTER
Int.Cl.  G06F16/35(2019.01)i

According to International Patent Classification (IPC) or to both national classification and IPC

B.  FIELDS SEARCHED

Minimum documentation searched (classification system followed by classification symbols)
Int.Cl.  G06F16/35

| Documentation searched other than minimum documentation to the extent that such documents are included in the fields searched | |
| --- | --- |
| Published examined utility model applications of Japan | 1922–1996 |
| Published unexamined utility model applications of Japan | 1971–2019 |
| Registered utility model specifications of Japan | 1996–2019 |
| Published registered utility model applications of Japan | 1994–2019 |

Electronic data base consulted during the international search (name of data base and, where practicable, search terms used)

C.  DOCUMENTS CONSIDERED TO BE RELEVANT

| Category* | Citation of document, with indication, where appropriate, of the relevant passages | Relevant to claim No. |
| --- | --- | --- |
| A | JP 2005-208782 A (FUJI XEROX CO., LTD.) 04 August 2005, paragraphs [0070]-[0073], [0099]-[0109] (Family: none) | 1-10 |
| A | WO 2017/199445 A1 (FRONTEO HEALTHCARE, INC.) 23 November 2017, paragraphs [0022]-[0032] (Family: none) | 1-10 |

☒  Further documents are listed in the continuation of Box C.     ☐  See patent family annex.

| | |
| --- | --- |
| * Special categories of cited documents:<br>"A" document defining the general state of the art which is not considered to be of particular relevance<br>"E" earlier application or patent but published on or after the international filing date<br>"L" document which may throw doubts on priority claim(s) or which is cited to establish the publication date of another citation or other special reason (as specified)<br>"O" document referring to an oral disclosure, use, exhibition or other means<br>"P" document published prior to the international filing date but later than the priority date claimed | "T" later document published after the international filing date or priority date and not in conflict with the application but cited to understand the principle or theory underlying the invention<br>"X" document of particular relevance; the claimed invention cannot be considered novel or cannot be considered to involve an inventive step when the document is taken alone<br>"Y" document of particular relevance; the claimed invention cannot be considered to involve an inventive step when the document is combined with one or more other such documents, such combination being obvious to a person skilled in the art<br>"&" document member of the same patent family |

| Date of the actual completion of the international search<br>02 July 2019 (02.07.2019) | Date of mailing of the international search report<br>09 July 2019 (09.07.2019) |
| --- | --- |
| Name and mailing address of the ISA/<br>Japan Patent Office<br>3-4-3, Kasumigaseki, Chiyoda-ku,<br>Tokyo 100-8915, Japan | Authorized officer<br><br>Telephone No. |

Form PCT/ISA/210 (second sheet) (January 2015)

**INTERNATIONAL SEARCH REPORT**

International application No.

PCT/JP2019/017191

C (Continuation).   DOCUMENTS CONSIDERED TO BE RELEVANT

| Category* | Citation of document, with indication, where appropriate, of the relevant passages | Relevant to claim No. |
|---|---|---|
| A | 横田 慎一郎、遠藤 美代子、平松 達雄、野口 貴史、美代 賢吾、大江 和彦，電子カルテデータを利用した後ろ向きコホートによる患者転倒リスク予測式の構築・評価・実装手法，医療情報学，第 34 巻，第 3 号，一般社団法人日本医療情報学会，29 September 2014, (YOKOTA, Shinichiro, ENDO, Miyoko, HIRAMATSU, Tatsuo, NOGUCHI, Takashi, MIYO, Kengo, OHE, Kazuhiko, "Construction, Evaluation, and Implementation of a Formula Predicting a Patient's Fall Risk Based on a Historical Cohort Study using Electronic Medical Records (EMR) Data", Japan Journal of Medical Informatics, vol. 34, no. 3, Japan Association for Medical Informatics) | 1-10 |
| A | JP 2018-32213 A (SHARP CORP.) 01 March 2018, paragraphs [0033], [0035], [0041]-[0045] (Family: none) | 1-10 |

Form PCT/ISA/210 (continuation of second sheet) (January 2015)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 5977898 B

**Non-patent literature cited in the description**

- Distributed Representations of Sentences and Documents. **QUOC LE ; TOMAS MIKOLOV.** Proceedings of the 31st International Conference on Machine Learning Held in Bejing. Google Inc, 22 June 2014